(19) Europäisches Patentamt
European Patent Office
Office européen des brevets

(11) EP 1 118 607 A1

(12) EUROPEAN PATENT APPLICATION

(43) Date of publication:
25.07.2001 Bulletin 2001/30

(51) Int Cl.[7]: **C07C 67/38**, C07C 69/533, C07C 67/37

(21) Application number: 00200208.7

(22) Date of filing: 19.01.2000

(84) Designated Contracting States:
**AT BE CH CY DE DK ES FI FR GB GR IE IT LI LU MC NL PT SE**
Designated Extension States:
**AL LT LV MK RO SI**

(71) Applicants:
- **DSM N.V.**
**6411 TE Heerlen (NL)**
- **E.I. DUPONT DE NEMOURS AND COMPANY**
**Wilmington, Delaware 19898 (US)**

(72) Inventors:
- **Schaffrath, Heike**
**52064 Aachen (DE)**
- **Baur, Henricus Anna Christiaan**
**6049 BE Roermond (NL)**
- **van Haaren, Johannes Antonius Egbertus Henricus**
**5628 ZT Eindhoven (NL)**
- **Sielcken, Otto Erik**
**6136 BG Sittard (NL)**
- **Haasen, Nicolaas Franciscus**
**6141 MB Sittard (NL)**

(74) Representative: **Kleiborn, Paul Erik et al**
**DSM Patents & Trademarks**
**Office Geleen**
**P.O. Box 9**
**6160 MA Geleen (NL)**

# (54) Process for the carbonylation of butadiene and/or at least one butadiene derivative

(57) Process for the carbonylation of butadiene and/or at least one butadiene derivative in the presence of water, an alkyl iodide and a rhodium containing catalyst system, wherein the carbonylation is performed in the presence of a carboxylic acid and the amount of water is higher than 0.4 wt.% and less than or equal to 5 wt.% (relative to the total liquid reaction mixture including the solvent(s), catalyst(s), promotor(s) and reactants).

EP 1 118 607 A1

## Description

The invention relates to a process for the carbonylation of butadiene and/or at least one butadiene derivative in the presence of water, an alkyl iodide and a rhodium containing catalyst system.

A carbonylation reaction according to this invention means every reaction between an unsaturated substrate and carbon monoxide, in which an acid or ester compound is obtained.

Example 16 of US-A-4622423 describes the preparation of 3-pentenoic acid by carbonylation of butadiene using a rhodium carbonylation catalyst system promoted with methyl iodide in the presence of water and methylene chloride solvent. Acetic acid in aqueous solution is said to be undesirable.

A disadvantage of this process is the relatively low level of catalyst activity. The turnover number (as a measure of catalyst activity) in example 16 is only 73 mol 3-pentenoic acid/mol rhodium (after 5 hours reaction time). The turnover frequency (turnover number per time unit) is 14.6 per hour. This low level of activity requires high catalyst concentration, long reaction times, high reactor pressures and/or high temperatures to obtain substantial reaction rates and conversions. This is disadvantageous because the larger the required process equipment becomes.

The object of the present invention is to provide a process for carbonylating butadiene and/or butadiene derivative in which the catalyst activity is higher than is achieved in the process according to US-A-4622423.

This object is achieved in that the carbonylation is performed in the presence of a carboxylic acid and the amount of water is higher than 0.4 wt.% and less than or equal to 5 wt.% (relative to the total liquid reaction mixture including the solvent(s), catalyst(s), promotor(s) and reactants).

It has been found that with the process according to the present invention the catalyst activity is considerably improved.

According to US-A-4788334 the use of acetic acid, trimethyl acetic acid or benzoic acid has a beneficial effect on the rate of carbonylation of hexene-1 or methyl-4-pentenoate in the presence of methyl iodide and a rhodium containing catalyst system and using methylene chloride as solvent. The turnover number in example 1 (as calculated above for example 16 of US-A-4622423) is however only 79 mol n-heptanoic acid/ mol rhodium (after 6.5 hours reaction time). The turnover frequency is thus only 12.1 per hour.

Suitable carboxylic acids are aliphatic $C_2$-$C_{20}$ monocarboxylic acids, aliphatic $C_4$-$C_{20}$ dicarboxylic acids, benzoic acid, $C_1$-$C_3$ alkyl substituted benzoic acids and mixtures thereof. Preferred carboxylic acids are aliphatic $C_2$-$C_{20}$ monocarboxylic acids, $C_4$-$C_7$ dicarboxylic acids, benzoic acid and mixtures thereof. Examples are acetic acid, propionic, butyric, 2-methylbutyric, valeric, caproic, crotonic acid, pentenoic and hexenoic acid and mixtures thereof. Preferred acids are aliphatic $C_2$-$C_9$ monocarboxylic acids. The most preferred acid is pentenoic acid. It has been found that when the carbonylation according to the invention is performed in the presence of pentenoic acid solvent, the separation of the rhodium catalyst and the pentenoate ester carbonylation product can easily be performed using for example vacuum distillation. The fact that in this preferred embodiment of the invention the separation of pentenoate ester and rhodium catalyst can be performed in a vacuum distillation unit can be regarded as a major advantage. Another reason why the use of pentenoic acid solvent is advantageous is that, if the process according to the present invention is performed in the presence of an alcohol, pentenoic acid reacts with the alcohol to the desired alkyl pentenoate ester. Furthermore, due to the presence of small amounts of water, pentenoic acid will be formed in a sufficient amount along with the pentenoate ester.

The amounts of reactants specified herein should preferably be maintained substantially during the whole process. By preference, the term substantially during the whole process means more than 90% of the process expressed in residence time.

The amount of carboxylic acid in the process of the invention is preferably higher than 0.5 wt.% and more preferably higher than 1 wt.%.

The amount of water in the process of the invention is preferably higher than 0.5 wt.%, more preferably higher than 1 wt.%. The concentration of water is preferably lower than 4.5 wt.% and more preferably lower than 2.5 wt.%. This water concentration is preferably present in the solution at the beginning of the reaction.

The molar ratio of alkyl iodide to rhodium is generally in the range of between 1:1 and 500:1. The molar ratio of alkyl iodide to rhodium is preferably greater than 2:1 and more preferably greater than 10:1.

When the carbonylation is performed in the presence of only water as a hydroxy group containing reactant, the product will be pentenoic acid. In case an alcohol is also present or formed during the reaction, a pentenoate ester product will be formed.

The pentenoic acid will be obtained as a mixture of 2-, 3- and 4-pentenoic acid. The pentenoate ester will be obtained as a mixture of 2-, 3- and 4-pentenoate ester.

Non-limitative examples of alcohols include the group of $C_1$ to $C_{10}$ alkyl alcohols, including methanol, ethanol, 1-propanol, 2-propanol, the butanols, pentanols and hexanols. Preferably, methanol or ethanol is used. The alkyl group of the alkyl pentenoate ester corresponds to the alcohol used.

In the present application, the term butadiene derivatives means those compounds which yield pentenoate

ester or pentenoic acid as the major product when carbonylated with the process according to the invention.

Suitable butadiene derivatives include the allylic butenols, allylic butenol esters, allylic butenyl ethers and/or allylic butenyl iodides.

The allylic butenol preferably is 1-butene-3-ol, 2-butene-1-ol or a mixture thereof.

Suitable allylic butenyl ethers and allylic butenol esters respectively are of the following formula (1) :

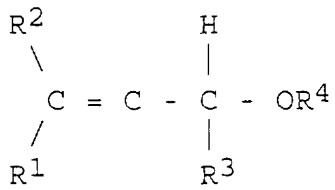

(1)

in which one of the groups $R^1$, $R^2$ and $R^3$ is methyl and the other two groups are H and $R^4$ is a $C_1$-$C_{10}$ alkyl group and

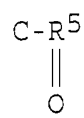

respectively. $R^5$ is a $C_1$-$C_{10}$ alkyl group or alkenyl group. Non-limitative examples of allylic butenyl ethers are 1-methoxy-2-butene, 3-methoxy-1-butene, 1-ethoxy-2-butene, 3-ethoxy-1-butene. Non-limitative examples of allylic butenol esters are crotyl acetate, crotyl propionate, crotyl valerate, crotyl adipate, crotyl-2-pentenoate, crotyl-3-pentenoate, crotyl-4-pentenoate, 1-butene-3-acetate, 1-butene-3-propionate, 1-butene-3-valerate, 1-butene-3-adipate, 1-butene-3-pentenoate. Preferred allylic butenol esters are crotyl acetate, crotyl valerate, crotyl-2-pentenoate, crotyl-3-pentenoate and/or crotyl-4-pentenoate. Most preferred allylic butenol esters are crotyl acetate, crotyl-2-pentenoate, crotyl-3-pentenoate and/or crotyl-4-pentenoate.

For purpose of the present invention, potential starting materials are mixtures of allylic butenols, allylic butenyl ether and/or allylic butenol esters or mixtures that produce the allylic butenols, allylic butenyl ether or allylic butenol esters in situ.

The allylic butenols can simply be prepared by hydrogenation of allylic butenal. The allylic butenols can also simply be prepared by reaction of butadiene with water as described in, for example, US-A-4645863. Esters of allylic butenols can be made by a method known for a man skilled in the art, for example, by reaction of the allylic butenols or of butadiene with the carboxylic acid

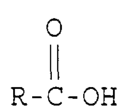

as described in, for example, US-A-4645863. Allylic butenyl ethers can also be made by a method known for a man skilled in the art, for example by reaction of butadiene with a $C_1$-$C_{10}$ alkyl alcohol as described in, for example, US-A-4343120 and US-A-4590300.

In one embodiment of the invention the carbonylation involves reacting butadiene, an allylic butenol and/or an allylic butenol ester and only water as hydroxy-containing compound to 3-pentenoic acid.

In another and preferred embodiment of the invention the carbonylation involves reacting butadiene and a C1-C10 alkyl alcohol to the corresponding alkyl-3-pentenoate.

In yet another and preferred embodiment of the invention an alkoxybutene is carbonylated to the corresponding alkyl-3-pentenoate. With an alkoxybutene is meant a compound according to formula (1) in which one of the groups $R^1$, $R^2$ and $R^3$ is methyl and the other two groups are H and $R^4$ is a $C_1$-$C_{10}$ alkyl group. Preferably, a mixture of 3-methoxy-1-butene, trans-1-methoxy-2-butene and cis-1-methoxy-2-butene is the starting alkoxybutene.

These preferred embodiments are advantageous if the carbonylation reaction product would be used directly in the hydroformylation to a terminal 5-formylvalerate ester with for example a rhodium-based catalyst system, such

as for example described in WO-A-9733854, because an intermediate reaction step for converting 3-pentenoic acid into the corresponding 3-pentenoate ester would not be necessary.

The rhodium can be present in the catalyst system in the form of a heterogeneous or homogeneous rhodium compound. Homogeneous systems are preferred. Since rhodium forms complexes with the iodide compound, the choice of the initial rhodium compound is in general not critical. Among the materials which can be employed as the source of the rhodium compound are rhodium metal, rhodium salts, rhodium oxides, rhodium carbonyl compounds, organorhodium compounds, coordination compounds of rhodium, and mixtures thereof. Specific examples of such materials include, but are not limited to, rhodium (III)chloride and its hydrates, $RhI_3$, $Rh(CO)_2I_3$, $Rh(CO)I_3$, rhodium(III) nitrate trihydrate, $Rh_4(CO)_{12}$, $[Rh(CO)_2I]_2$, $Rh_6(CO)_{16}$, $Rh(acac)_3$, $Rh(CO)_2(acac)$, $Rh(C_2H_4)_2(acac)$, $Rh(CO)_2$acetate, $[Rh(C_2H_4)_2Cl]_2$, $[Rh(CO)_2Cl]_2$, Rh(COD)(acac), $[Rh(COD)Cl]_2$, $RhCl(CO)(PPh_3)_2$, $Rh_2[O_2C(CH_2)_6CH_3]_4$ and $Rh_2$(acetate)$_4$, where acac is acetylacetonate, COD is 1,5-cyclooctadiene and Ph is phenyl. Examples of sources of rhodium catalyst include rhodium(I) compounds such as $Rh(CO)_2(acac)$, $[Rh(CO)_2I]_2$, Rh(COD)(acac), and rhodium (III) iodide compounds such as $RhI_3$, $Rh(CO)_2I_3$. Supported rhodium compounds can also be used as a source of the rhodium catalyst. Examples of supported rhodium compounds are Rh/C, Rh/alumina and rhodium on polymeric supports, e.g. rhodium on polyvinylpyridine and rhodium on polyvinylpyrrolidon.

Suitable concentrations of rhodium in the reaction medium are in the range of 0.005-0.50 % by weight of rhodium metal based on the weight of the reaction medium. Preferably, the concentration of rhodium is in the range of 0.01-0.20 wt.%.

The process according to the present invention is performed in the presence of a rhodium containing catalyst, which may be pre-formed or generated in situ, and an alkyl iodide compound. Suitable alkyl iodide compounds have alkyl moieties corresponding to the alkyl moiety of the alkyl alcohol reactant (if present). Preferred alkyl iodides are $C_1$-$C_{10}$ alkyl iodides, more preferred alkyl iodides are $C_1$-$C_6$ alkyl iodides and yet more preferred alkyl iodides are $C_1$-$C_4$ alkyl iodides. Examples of these alkyl iodides are methyl iodide, iodoethane, 2-iodobutane, 1,4-di-iodobutane, 2-iodopropane, 1-iodopropane and iodoheptane. The most preferred alkyl iodide is methyl iodide.

For purpose of the present invention, mixtures that produce the alkyl iodide compound in situ are equivalent alkyl iodide compounds.

Generally, the concentration of alkyl iodide compound is between 0.03 wt.% and 50 wt.% based upon the weight of the reaction medium, preferably between 0.1 and 5 wt.%.

The temperature of the reaction is generally between 40°C and 250°C, but best rates and yields are achieved between 100°C and 160°C. Above 160°C, yields to pentenoate acid and/or ester are reduced. Below 100°C, reaction rates become too slow for practical commercial exploitation.

The carbon monoxide partial pressure applied during the carbonylation reaction is in the range between 0.5 and 20 MPa, preferably between 1.5 and 15 MPa.

The invention will be elucidated with the following non-limiting examples. The selectivity, turnover number, and turnover frequency mentioned in the examples are defined as follows:

Selectivity to pentenoic acid and/or methyl-3-pentenoate =

obtained amount of pentenoic acid and/or methyl-3-pentenoate (mol)*

100%/converted amount of substrate (mol).

Turnover number = obtained amount of pentenoic acid

and/or methyl-3-pentenoate (mol)/amount of rhodium (mol) .

Turnover frequency = Turnover number/reaction time.

Example 1 : Carbonylation of butadiene

A 150 ml mechanically stirred Hastelloy-C autoclave was charged with 0.13 g Rh(acac) $(CO)_2$ (0.5 mmol), 30.95 g methyl iodide (218 mmol, 32.6 wt.%), 5.93 g methanol (185 mmol) , 0.15 g water (8 mmol, 0.16 wt.%), 8.3 g butadiene (154 mmol) and 50 ml methyl acetate. The solution was heated to a temperature of 130°C under a CO-pressure of 5 MPa. The reaction just started significantly when 2.46 g water (137 mmol, 2.6 wt.%) were injected. At this time, 0.9 g acetic acid had been formed (15 mmol, 0.95 wt.%). During the run the total pressure was kept between

4.5 and 5 MPa by feeding carbon monoxide from a reservoir at intervals. The molar ratio of iodide to rhodium was 436.

After 2.0 hours, 26.6% (41 mmol) methyl-3-pentenoate (M3P) and 18.2% (28 mmol) 3-pentenoic acid (3-PA) had been formed. The selectivity to (M3P+3-PA) was 83%.The turnover number was 138 in 2 hours. The turnover frequency was 69/hour.

Example 2 : Carbonylation of butadiene

A 150 ml mechanically stirred Hastelloy-C autoclave was charged with 0.12 g Rh (acac) $(CO)_2$ (0.47 mmol), 9.04 g methyl iodide (63.7 mmol, 11.2 wt.%), 7.57 g methanol (237 mmol) , 2.15 g water (119.5 mmol, 2.67 wt.%) and 50 g valeric acid (62.4 wt.%). The solution was heated to a temperature of 130°C with agitation (1000 rpm) under an initial pressure of 1 Mpa carbon monoxide. The reaction was initiated by injection of 11.3 g (209 mmol) butadiene and adjusting the total pressure to 5 Mpa. During the run the total pressure was kept between 4 and 5 Mpa by feeding carbon monoxide from a reservoir at intervals.ly when 2.46 g water (137 mmol) were injected. At this time, 0.9 g acetic acid had been formed (15 mmol). During the run the total pressure was kept between 4.5 and 5 MPa by feeding carbon monoxide from a reservoir at intervals.

After 100 minutes, 5% methyl-3-pentenoate (10.45 mmol) and 15 % 3-pentenoic acid (31.35 mmol) were obtained. The turnover number was 89 and the turnover frequency was 89 per hour.

Comparing Example 1 and 2 with Example 16 of US-A-4622423 shows that the catalyst activity in the process of the present invention is considerably higher, even after less reaction time and at lower catalyst concentration.

Example 3 : Carbonylation of 3-methoxy-1-butene

A 150 ml mechanically stirred Hastelloy-C autoclave was charged with 0.127 g (0.5 mmol) Rh(acac) $(CO)_2$, 8.9 g (63 mmol, 10.3 wt.%) methyl iodide, 1.87 g (104 mmol, 2.17 wt.%) water and 70 g valeric acid (81.2 wt.%). The solution was heated to a temperature of 130°C under an initial CO-pressure of 1 MPa. The reaction was initiated by injection of 5.66 g (65 mmol) 3-methoxy-1-butene. The pressure was adjusted to 5 MPa. During the run the total pressure was kept between 4.5 and 5 MPa by feeding carbon monoxide from a reservoir at intervals. The molar ratio of iodide to rhodium was 126. After 30 minutes the reaction was stopped and the reaction products were analyzed by gas chromatographic methods.

After 30 min, 49% M3P (31.7 mmol) and 50% 3-PA (32.5 mmol) were obtained at an overall selectivity to (M3P+3-PA) of 99%. The turnover number was 131 after 0.5 hour. The turnover frequency was 262 per hour.

Example 4 : Carbonylation of 1-methoxy-2-butene

A 150 ml mechanically stirred Hastelloy-C autoclave was charged with 0.13 g ( 0.5 mmol) Rh(acac)$(CO)_2$, 7.9 g ( 55.6 mmol, 11.6 wt.%) methyl iodide, 1.36 g (76 mmol, 2 wt%) water and 53.5 g (78.7 wt.%)valeric acid. The solution was heated to a temperature of 130°C under an initial CO-pressure of 1 MPa. The reaction was initiated by injection of 5.44 g ( 63 mmol) 1-methoxy-2-butene. The pressure was adjusted to 5 MPa. During the run the total pressure was kept between 4.5 and 5 MPa by feeding carbon monoxide from a reservoir at intervals. The molar ratio of iodide to rhodium was 111. After 15 minutes the reaction was stopped and the reaction products were analyzed by gas chromatographic methods.

After 15 min, 89% (M3P+3-PA) (56.1 mmol) were obtained at an overall selectivity of 93 The turnover number was 112 in 15 minutes. The turnover frequency was 448 per hour.

Comparative Experiment A

In comparison to example 2, this experiment was started in methyl valerate instead of valeric acid, thus without carboxylic acid.

A 150 ml mechanically stirred Hastelloy-C autoclave was charged with 0.117 g (0.45 mmol) Rh(acac) $(CO)_2$, 8.0 g (56 mmol) methyl iodide, 1.15 g (88 mmol, 1.7%) water and 50 g methyl valerate. The solution was heated to a temperature of 130°C under an initial CO-pressure of 1 MPa. The reaction was initiated by injection of 7.6 g of a 1/1 mixture of 3-methoxy-1-butene and 1-methoxy-2-butene (88 mmol). The pressure was adjusted to 5 MPa. During the run the total pressure was kept between 4.5 and 5 MPa by feeding carbon monoxide from a reservoir at intervals.

In general, the reaction rate increased with an increase of carboxylic acid content (formed from deesterification). At 0 wt.% carboxylic acid, no reaction was observed. At 0.5 wt.% carboxylic acid the turnover frequency raised to 13 per hour (7% (M3P and 3-PA were formed). The turnover frequency further raised with the increased amount of carboxylic acid. After 90 min, when 3 wt.% carboxylic acid was present, 48% (M3P+3-PA) were formed, which corresponds to a turnover number of 94. The turnover frequency was 63 per hour.

This demonstrates the enhancing effect of the carboxylic acid, which is formed here in situ. Wheras high turnover frequency's were obtained in the initial presence of both water and acid (see examples 2 and 3), the turnover frequency increases here in relation to the acid formed.

Comparative Experiment B

In comparison to example 2, this experiment was carried out with methanol instead of water.

A 150 ml mechanically stirred Hastelloy-C autoclave was charged with 120 g (0.47 mmol) Rh(acac) $(CO)_2$, 8.48 g (60 mmol) methyl iodide, 2.66 g (83 mmol) methanol and 48 g valeric acid. The solution was heated to a temperature of 130°C under an initial CO-pressure of 1 MPa. The reaction was initiated by injection of 5.56 g (65 mmol) 3-methoxy-1- butene. The pressure was adjusted to 5 MPa. During the run the total pressure was kept between 4.5 and 5 MPa by feeding carbon monoxide from a reservoir at intervals.

No reaction was observed at water concentration below 0.4 wt.%. The reaction seriously started at 0.4 wt.% water. After 25 minutes, when 0.7 wt.% water was formed, the turnover frequency was 28 per hour. After 40 minutes, when 0.9 wt.% water was formed, the turnover frequency was 38 per hour. After 60 minutes, when 1.1 wt.% water was formed, the turnover frequency was 49 per hour. After 90 minutes, when 1.5 wt.% water was formed, the turnover frequency was 57 per hour. The reaction rate showed an increase with an increase of water formation from esterification. After 90 min, 63% (M3P+3-PA) were formed (productivity of 0.40 mol (M3P+3-PA)/l/h).

Example 5: Carbonylation of crotylalcohol (2-butene-1-ol)

A 150 ml mechanically stirred Hastelloy-C autoclave was charged with 0.20 g (0.77 mmol) Rh(acac)$(CO)_2$, 4.0 g (28 mmol, 8.2 wt.%) methyl iodide, 1.6 g (90 mmol, 3.3 wt.%) water and 36 g nonanoic acid (74.2 wt.%). The solution was heated to a temperature of 110°C under an initial CO-pressure of 1 MPa. The reaction was initiated by injection of 6.5 g (90 mmol) crotylalcohol. The pressure was adjusted to 5 MPa. During the run the total pressure was kept between 4.5 and 5 MPa by feeding carbon monoxide from a reservoir at intervals. The molar ratio of iodide to rhodium was 36. After 1 hour the reaction was stopped and the reaction products were analyzed by gas chromatographic methods.

After 60 min, crotyl alcohol was converted to 98%. The selectivity to 3PA was 95 The turnover number was 109 in 1 hour. The turnover frequency was thus 109/hour.

## Claims

1. Process for the carbonylation of butadiene and/or at least one butadiene derivative in the presence of water, an alkyl iodide and a rhodium containing catalyst system, characterized in that, the carbonylation is performed in the presence of a carboxylic acid and the amount of water is higher than 0.4 wt.% and less than or equal to 5 wt.% (relative to the total liquid reaction mixture including the solvent(s), catalyst(s), promotor(s) and reactants).

2. Process according to claim 1, characterized in that, said carboxylic acid is an aliphatic $C_2$-$C_{20}$ monocarboxylic acid.

3. Process according to claim 2, characterized in that, said carboxylic acid is pentenoic acid.

4. Process according to any one of claims 1-3, characterized in that, the concentration of water is between 0.5 and 2.5 wt.%.

5. Process according to any one of claims 1-4, characterized in that, butadiene is reacted with a C1-C10 alkyl alcohol to the corresponding alkyl-3-pentenoate.

6. Process according to any one of claims 1-4, characterized in that, the butadiene derivative is an alkoxybutene.

7. Process according to claim 6, characterized in that, said alkoxybutene is a mixture of 3-methoxy-1-butene, trans-1-methoxy-2-butene and cis-1-methoxy-2-butene.

8. Process according to any one of claims 1-7, characterized in that, the alkyl iodide is methyl iodide.

European Patent Office

# EUROPEAN SEARCH REPORT

Application Number
EP 00 20 0208

**DOCUMENTS CONSIDERED TO BE RELEVANT**

| Category | Citation of document with indication, where appropriate, of relevant passages | Relevant to claim | CLASSIFICATION OF THE APPLICATION (Int.Cl.7) |
|---|---|---|---|
| A | EP 0 728 732 A (DSM N.V.) 28 August 1996 (1996-08-28)<br>* page 3, line 19 - line 40 *<br>* page 4, line 16 - line 33 *<br>* page 4, line 43 - line 48 *<br>* page 8; examples 18,19 *<br>* page 10 - page 11; claims *<br>----- | 1 | C07C67/38<br>C07C69/533<br>C07C67/37 |

TECHNICAL FIELDS SEARCHED (Int.Cl.7)
C07C

The present search report has been drawn up for all claims

| Place of search | Date of completion of the search | Examiner |
|---|---|---|
| THE HAGUE | 24 May 2000 | Kinzinger, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 00 20 0208

This annex lists the patent family members relating to the patent documents cited in the above-mentioned European search report.
The members are as contained in the European Patent Office EDP file on
The European Patent Office is in no way liable for these particulars which are merely given for the purpose of information.

24-05-2000

| Patent document cited in search report | Publication date | Patent family member(s) | Publication date |
|---|---|---|---|
| EP 728732 A | 28-08-1996 | CA 2169892 A | 23-08-1996 |
| | | CN 1138021 A | 18-12-1996 |
| | | JP 8245510 A | 24-09-1996 |
| | | SG 44551 A | 22-06-1999 |
| | | US 5672732 A | 30-09-1997 |

EPO FORM P0459

For more details about this annex : see Official Journal of the European Patent Office, No. 12/82